# EUROPEAN PATENT SPECIFICATION

(11) **EP 0 719 140 B1**
(45) Date of publication and mention of the grant of the patent: **31.10.2007**
(21) Application number: 94929204.9
(22) Date of filing: 14.09.1994
(51) Int. Cl.: A61K 31/41, A61K 31/42, A61K 31/425, A61K 31/44, A61K 31/495, A61K 31/505

(54) **USE OF THIAZOLIDINEDIONES TO PREVENT OR DELAY ONSET OF NIDDM**
VERWENDUNG VON THIAZOLIDINDIONEN ZUR VORBEUGUNG ODER VERZÖGERUNG DES AUFTRETENS DES NICHTINSULINABHÄNGIGEN DIABETES MELLITUS (NIDDM)
UTILISATION DE THIAZOLIDINEDIONES POUR PREVENIR OU RETARDER L'APPARITION DU DIABETE SUCRE NON INSULINODEPENDANT

(30) Priority: 15.09.1993 US 122251; 23.08.1994 US 293899
(43) Date of publication of application: 03.07.1996
(60) Divisional application: 05027983.5
(73) Proprietor: Daiichi Sankyo Company, Limited, Chuo-ku Tokyo (JP)
(72) Inventor: OLEFSKY, Jerrold, Solano Beach, CA 92075 (US); ANTONUCCI, Tammy, Mequon, WI 53092 (US); LOCKWOOD, Dean, Ann Arbor, MI 48103 (US); NORRIS, Rebecca, Kewadin, MI 49648 (US)
(74) Representative: Gibson, Christian John Robert
(86) International application number: PCT/US1994/010389
(87) International publication number: WO 1995/007694

(56) References cited:
- EP-A- 0 332 332
- EP-A- 0 419 035
- EP-A- 0 439 321
- EP-A- 0 528 734
- EP-A- 0 559 571
- WO-A-92/07838
- WO-A-92/07839
- WO-A-93/00343
- US-A- 4 287 200
- US-A- 4 572 912
- US-A- 4 687 777
- US-A- 4 873 255
- US-A- 4 897 405
- US-A- 5 061 717
- US-A- 5 132 317
- NOLAN, J. J. ET AL: "Metabolic effects of troglitazone in subjects with impaired glucose tolerance and insulin resistance." CLINICAL RESEARCH, (1994) VOL. 42, NO. 1, PP. 88A. MEETING INFO.: JOINT MEETING OF THE WESTERN SOCIETY FOR CLINICAL INVESTIGATION, WESTERN SECTION OF THE AMERICAN FEDERATION FOR CLINICAL RESEARCH, WESTERN SOCIETY FOR PEDIATRIC RESEARCH, WESTERN REGIO, XP000981774
- FUJIWARA, TOSHIHIKO ET AL: "Characterization of new oral antidiabetic agent CS-045. Studies in KK and ob/ob mice and Zucker fatty rats" DIABETES (1988), 37(11), 1549-58, XP000981561
- IWAMOTO Y ET AL: "Effect of new oral antidiabetic agent CS - 045 on glucose tolerance and insulin secretion in patients with NIDDM." DIABETES CARE, (1991 NOV) 14 (11) 1083-6., XP000981534
- FUJITA T. ET AL: "Reduction of insulin resistance in obese and/or diabetic animals by 5-[4-(1-methylcyclohexylmethoxy)benzyl]-th iazolidine-2,4-dione ( ADD - 3878, U-63,287, Ciglitazone ), a new antidiabetic agent." DIABETES, (1983) 32/9 (804-810). CODEN: DIAEAZ, XP000981539
- SUGIYAMA, Y. ET AL: "Effects of pioglitazone on glucose and lipid metabolism in Wistar fatty rats" ARZNEIM.-FORSCH. (1990), 40(3), 263-7, XP000979419
- IKEDA, H. ET AL: "Effects of pioglitazone on glucose and lipid metabolism in normal and insulin resistant animals" ARZNEIM.-FORSCH. (1990), 40(2), 156-62, XP000979418

## Description

### FIELD OF THE INVENTION

The present invention pertains to a number of compounds which can be used in the manufacture of a medicament to treat impaired glucose intolerance in order to prevent or delay the onset of noninsulin-dependent diabetes mellitus (NIDDM). More specifically, the present invention involves in one embodiment administering to a patient certain known thiazolidinedione derivatives and related antihyperglycemic agents which reduce fasting insulin levels and return normal glucose tolerance to an individual, thus preventing or delaying the onset of NIDDM or complications resulting therefrom.

### BACKGROUND

Diabetes is one of the most prevalent chronic disorders worldwide with significant personal and financial costs for patients and their families, as well as for society. Different types of diabetes exist with distinct etiologies and pathogeneses. For example, diabetes mellitus is a disorder of carbohydrate metabolism, characterized by hyperglycemia and glycosuria and resulting from inadequate production or utilization of insulin.

Diabetes mellitus often develops from certain at risk populations, one such population is individuals with impaired glucose tolerance (IGT). Impaired glucose tolerance is a condition intermediate between frank, noninsulin-dependent diabetes mellitus and normal glucose tolerance in which the affected person's postprandial glucose response is abnormal as assessed by 2-hour postprandial plasma glucose levels. This IGT population progresses to a certain form of diabetes mellitus, specifically noninsulin-dependent diabetes mellitus (NIDDM).

NIDDM or otherwise referred to as Type II diabetes is the form of diabetes mellitus which occurs predominantly in adults in whom adequate production of insulin is available for use, yet a defect exists in insulin-mediated utilization and metabolism of glucose in peripheral tissues. It has been shown that for some people with diabetes a genetic predisposition results in a mutation in the gene(s) coding for insulin and/or the insulin receptor and/or insulin-mediated signal transduction factor(s), thereby resulting in ineffective insulin and/or insulin-mediated effects thus impairing the utilization or metabolism of glucose. The population with impaired glucose tolerance progresses to NIDDM at a rate of 5% to 10% of cases per year.

Failure to treat NIDDM can result in mortality due to cardiovascular disease and in other diabetic complications including retinopathy, nephropathy, and peripheral neuropathy. For many years treatment of NIDDM has involved a program aimed at lowering blood sugar with a combination of diet and exercise. Alternatively, treatment of NIDDM involved oral hypoglycemic agents, such as sulfonylureas alone or in combination with insulin injections. Recently, alpha-glucosidase inhibitors, such as acarbose, have been shown to be effective in reducing the postprandial rise in blood glucose (Lefevre, et al., Drugs 1992;44: 29-38). In Europe and Canada another treatment used primarily in obese diabetics is metformin, a biguanide.

In any event, what is required is a method of treating populations experiencing impaired glucose tolerance in order to prevent or delay the onset of NIDDM thereby bringing relief of symptoms, improving the quality of life, preventing acute and long-term complications, reducing mortality and treating accompanying disorders of those at risk for NIDDM. The methods of using the disclosed compounds for treating populations experiencing impaired glucose tolerance to prevent or delay the onset of NIDDM as taught herein meet these objectives.

Compounds useful for practicing the present invention, and methods of making these compounds are known. Some of these compounds are disclosed in WO 91/07107; WO 92/02520; WO 94/01433; WO 89/08651; JP Kokai 69383/92; U.S. Patent Nos. 4,287,200; 4,340,605; 4,438,141; 4,444,779; 4,461,902; 4,572,912; 4,687,777; 4,703,052; 4,725,610; 4,873,255; 4,897,393; 4,897,405; 4,918,091; 4,948,900; 5,002,953; 5,061,717; 5,120,754; 5,132,317; 5,194,443; 5,223,522; 5,232,925; and 5,260,445. The active compounds disclosed in these publications are useful as therapeutic agents for the treatment of diabetes, hyperglycemia, hypercholesterolemia, and hyperlipidemia. The disclosure of these publications are incorporated herein by reference in particular with respect to the active compounds disclosed therein, and methods of preparation thereof. These compounds are useful for the treatment of impaired glucose tolerance (IGI) in order to prevent or delay onset of NIDDM and complications resulting therefrom, in accordance with the present invention.

Fujita et al., Diabetes (1983), 32/9, pp.804-810 discloses the administration of a thiazolidinedione, ciglitazone, to beagle dogs in a trial that established that this compound impedes increase in blood glucose levels; the authors conclude that ciglitazone has potential in the treatment of patients who are obese and/or have type II diabetes.

Iwamoto et al., Diabetes Care (1991), vol. 14, no.11, pp. 1083-1086 and Fujiwara et al., Diabetes (1998), vol. 37, no. 11, pp. 1549-1558 disclose the effectiveness of troglitazone (CS-045) in improving glucose tolerance and insulin response in patients with NIDDM.

Ikeda et al., Arzneimittelforschung/Drug Research (1990), vol. 40(1), no. 2, pp. 156-162 discloses that, on the basis of tests performed in various animal models, pioglitazone can impede increases in blood glucose levels, making it of potential use in the treatment of patients who are obese and/or have NIDDM.

There is no disclosure in the above-identified references to use the compounds identified in this present application in the treatment of populations experiencing impaired glucose tolerance in order to prevent or delay the onset of NIDDM and complications resulting therefrom.

### SUMMARY OF THE INVENTION

The present invention provides the use of various known compounds for the manufacture of a medicament for the treatment of impaired glucose tolerance in order to prevent or delay the onset of NIDDM. It is known that persons with impaired glucose tolerance have a much higher rate of progression to NIDDM than persons with normal glucose tolerance. Saad, et al., New Engl J Med 1988; 319:1500-6. If impaired glucose tolerance can be normalized, it is likely that the progression to NIDDM will be delayed or prevented in this population.

Compounds useful for practicing the present invention reduce fasting insulin levels, improve insulin sensitivity, and return glucose tolerance to the normal range for many individuals. As agents having the aforementioned effects (in the return of glucose tolerance), the compounds of the following formulas are useful in prophylactically treating individuals to prevent or delay the onset of NIDDM.

Accordingly, the present invention is the use of compounds of formula II, as defined below, in the manufacture of a medicament for the treatment of impaired glucose tolerance to prevent or delay the onset of noninsulin-dependent diabetes mellitus: 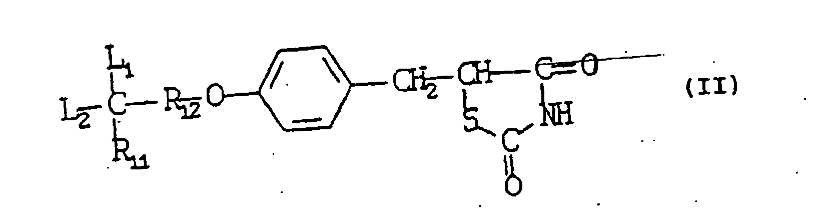 wherein R₁₁ is substituted or unsubstituted alkyl, alkoxy, cycloalkyl, phenylalkyl, phenyl, aromatic acyl group, a 5- or 6-membered heterocyclic group including 1 or 2 heteroatoms selected from the group consisting of nitrogen, oxygen, and sulfur, or a group of the formula 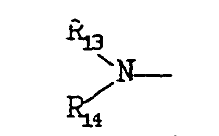 wherein R₁₃ and R₁₄ are the same or different and each is lower alkyl or R₁₃ and R₁₄ are combined to each other either directly or as interrupted by a heteroatom selected from the group consisting of nitrogen, oxygen, and sulfur to form a 5- or 6-membered ring;
wherein R₁₂ means a bond or a lower alkylene group; and
wherein L₁ and L₂ are the same or different and each is hydrogen or lower alkyl or L₁ and L₂ are combined to form an alkylene group; or a pharmaceutically acceptable salt thereof.

Referring to the general Formula II, the substituents may be any from 1 to 3 selected from nitro, amino, alkylamino, dialkylamino, alkoxy, halo, alkyl, or hydroxy, the aromatic acyl group may be benzoyl and naphthoyl. The alkyl group R₁₁ may be a straight chain or branched alkyl of 1 to 10 carbon atoms, such as methyl, ethyl, n-propyl, i-propyl, n-butyl, i-butyl, t-butyl, n-pentyl, i-pentyl, n-hexyl, n-heptyl, n-octyl, n-nonyl, and n-decyl; the cycloalkyl group R₁₁ may be a cycloalkyl group of 3 to 7 carbon atoms, such as cyclopropyl, cyclopentyl, cyclohexyl, and cycloheptyl; and the phenylalkyl group R₁₁ may be a phenylalkyl group of 7 to 11 carbon atoms such as benzyl and phenethyl. As examples of the heterocyclic group R₁₁ may be mentioned 5- or 6-membered groups each including 1 or 2 hetero-atoms selected from among nitrogen, oxygen, and sulfur, such as pyridyl, thienyl, furyl, thiazolyl, etc. When R₁₁ is 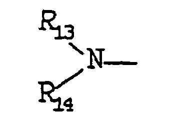 the lower alkyls R₁₃ and R₁₄ may each be a lower alkyl of 1 to 4 carbon atoms, such as methyl, ethyl, n-propyl, i-propyl, and n-butyl. When R₁₃ and R₁₄ are combined to each other to form a 5- or 6-membered heterocyclic group as taken together with the adjacent N atom, this heterocyclic group may further include a heteroatom selected from among nitrogen, oxygen, and sulfur as exemplified by piperidino, morpholino, pyrrolidino, and piperazino.

The lower alkylene group R₁₂ may contain 1 to 3 carbon atoms and thus may be, for example, methylene, ethylene, or trimethylene. The bond R₁₂ is equivalent to the symbol "-", ".", or the like which is used in chemical structural formulas.

Specific examples of compounds of use in the present invention are given in the following list:
5-[4-[(1-methylcyclohexyl)methoxy]benzyl]thiadiazolidine-2,4-dione (ciglitazone); and
5-[4-[2-(5-ethylpyridin-2-yl)ethoxy]benzyl]thiazolidine-2,4-dione (pioglitazone.

As defined herein, "complications of NIDDM" is referred to as cardiovascular complications or several of the metabolic and circulatory disturbances that are associated with hyperglycemia, e.g., insulin resistance, hyperinsulinemia and/or hyperproinsulinemia, delayed insulin release, dyslipidemia, retinopathy, peripheral neuropathy, nephropathy, and hypertension.

The compounds of Formula (II) are capable of further forming pharmaceutically acceptable base salts.

The compounds of Formula (II) are capable of forming both pharmaceutically acceptable acid addition and/or base salts. All of these forms are within the scope of the present invention.

Pharmaceutically acceptable acid addition salts of the compounds of Formula (II) includes salts derived from non toxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate, n-methyl glucamine (see, for example, Berge S.M., et al., "Pharmaceutical Salts," Journal of Pharmaceutical Science 1977;66:1-19).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner or as above. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge S.M., et al., "Pharmaceutical Salts," Journal of Pharmaceutical Science 1977;66:1-19).

The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner or as above. The free acid forms differ from their respective salt forms somewhat in certain physical properties such as solubility in polar solvents, but otherwise the salts are equivalent to their respective free acid for purposes of the present invention.

Certain of the compounds of the present invention can exist in unsolvated forms as well as solvated forms, including hydrated forms. In general, the solvated forms, including hydrated forms, are equivalent to unsolvated forms and are intended to be encompassed within the scope of the present invention. Certain of the compounds of the present invention possess one or more chiral centers and each center may exist in different configurations. The compounds can, therefore, form stereoisomers. Although these are all represented herein by a limited number of molecular formulas, the present invention includes the use of both the individual, isolated isomers and mixtures, including racemates, thereof. Where stereospecific synthesis techniques are employed or optically active compounds are employed as starting materials in the preparation of the compounds, individual isomers may be prepared directly; on the other hand, if a mixture of isomers is prepared, the individual isomers may be obtained by conventional resolution techniques, or the mixture may be used as it is, without resolution.

Furthermore, the thiazolidene part of the compound of Formula (II) can exist in the form of tautomeric isomers. All of the tautomers are represented by Formula II) and are intended to be a part of the present invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection liquid preparations can be formulated in solution in aqueous polyethylene glycol solution.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsules, tablet, cachet, or lozenge itself, or it can be the appropriate number of any of these in packaged form.

The quantity of active component in a unit dose preparation may be varied or adjusted from 0.1 mg to 100 mg preferably 0.5 mg to 100 mg according to the particular application and the potency of the active component. The composition can, if desired, also contain other compatible therapeutic agents.

In therapeutic use in the treatment of at risk populations such as those with impaired glucose tolerance, to prevent or delay the onset of NIDDM and complications arising therefrom, the compounds utilized in the pharmaceutical methods of this invention are administered along with a pharmaceutically acceptable carrier at the initial dosage of about 0.01 mg to about 20 mg per kilogram daily. A daily dose range of about 0.01 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day, if desired.

## Claims

1. Use of a compound of formula (II), as defined below, in the manufacture of a medicament for the treatment of impaired glucose tolerance to prevent or delay the onset of noninsulin-dependent diabetes mellitus: 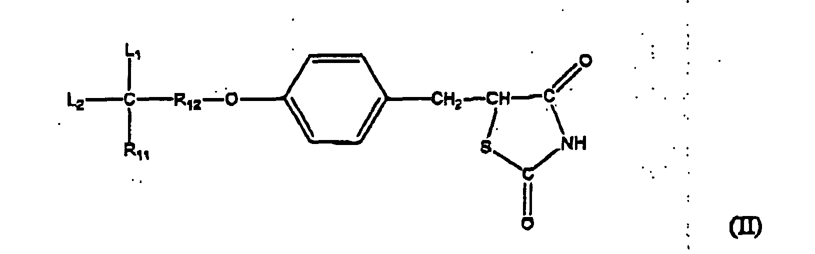 wherein R₁₁ is substituted or unsubstituted alkyl, alkoxy, cycloalkyl, phenylalkyl, phenyl, aromatic acyl group, a 5- or 6-membered heterocyclic group including 1 or 2 heteroatoms selected from the group consisting of nitrogen, oxygen, and sulfur, or a group of the formula 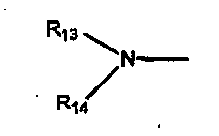 wherein R₁₃ and R₁₄ are the same or different and each is lower alkyl or R₁₃ and R₁₄ are combined to each other either directly or are interrupted by a heteroatom selected from the group consisting of nitrogen, oxygen, and sulfur to form a 5- or 6-membered ring;
R₁₂ means a bond or a lower alkylene group; and
L₁ and L₂ are the same or different and each is hydrogen or lower alkyl or L₁ and L₂ are combined to form an alkylene group, or a pharmaceutically acceptable salt theteof.

2. Use according to claim 1 of a compound of Formula (II) wherein the compound is 5-[4-[2-(5-ethylpyridin-2-yl)ethoxy]benzyl]thiadiazolidine-2,4-dione (pioglitazone).

3. Use according to claim 1 of a compound of Formula (II) wherein the compound is 5-[4-[(1-methylcyclohexyl)methoxy]benzyl]thiadiazolidine-2,4-dione (ciglitazone).

## Patentansprüche

1. Verwendung einer Verbindung der Formel (II), wie nachstehend definiert, bei der Herstellung eines Arzneimittels zur Behandlung der beeinträchtigten Glucosetoleranz zur Verhinderung oder Verzögerung des Auftretens des nicht insulinabhängigen Diabetes mellitus: 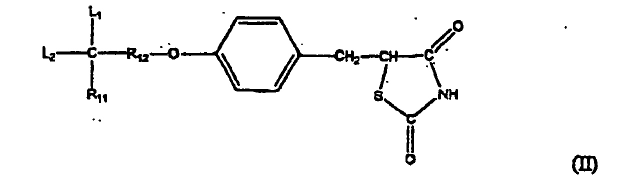 worin R₁₁ Folgendes darstellt: eine substituierte oder nicht substituierte Alkyl-, Alkoxy-, Cycloalkyl-, Phenylalkyl-, Phenyl- oder aromatische Acylgruppe, eine 5- oder 6-gliedrige heterocyclische Gruppe, die 1 oder 2 Heteroatom(e) einschließt, das/die aus der Gruppe ausgewählt ist/sind, bestehend aus Stickstoff, Sauerstoff und Schwefel, oder eine Gruppe der Formel 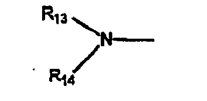 worin R₁₃ und R₁₄ gleich oder verschieden sind und jedes ein niederes Alkyl darstellt oder R₁₃ und R₁₄ entweder direkt miteinander kombiniert sind oder durch ein Heteroatom unterbrochen sind, das aus der Gruppe ausgewählt ist, bestehend aus Stickstoff, Sauerstoff und Schwefel zur Bildung eines 5- oder 6-gliedrigen Rings;
worin R₁₂ eine Bindung oder eine niedere Alkylengruppe bedeutet; und
worin L₁ und L₂ gleich oder verschieden sind und jedes Wasserstoff oder ein niederes Alkyl darstellt oder L₁ und L₂ zur Bildung einer Alkylengruppe oder eines pharmazeutisch verträglichen Salzes davon kombiniert sind.

2. Verwendung nach Anspruch 1 einer Verbindung der Formel (II), worin die Verbindung 5-[4-[-2-(5-Ethylpyridin-2-yl)ethoxy]benzyl]thiadiazolidin-2,4-dion (Pioglitazon) darstellt.

3. Verwendung nach Anspruch 1 einer Verbindung der Formel (II), worin die Verbindung 5-[4-[(1-Methylcyclohexyl)methoxy]benzyl]thiadiazolidin-2,4-dion (Ciglitazon) darstellt.

## Revendications

1. Utilisation d'un composé de formule (II), telle que définie ci-après, lors de la fabrication d'un médicament pour le traitement d'une tolérance au glucose détériorée, pour prévenir ou retarder l'apparition d'un diabète sucré non insulino-dépendant: 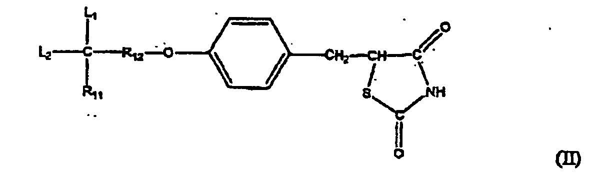 dans laquelle R₁₁ est un groupe alkyle, alcoxy, cycloalkyle, phénylalkyle, phényle, acyle aromatique, substitué ou non substitué, un groupe hétérocyclique à 5 ou 6 chaînons incluant 1 ou 2 hétéroatomes choisis dans le groupe constitué par l'azote, l'oxygène et le soufre, ou un groupe de formule 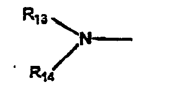 dans laquelle R₁₃ et R₁₄ sont identiques ou différents et chacun est un alkyle inférieur ou R₁₃ et R₁₄ sont combinés l'un à l'autre soit directement soit sont interrompus par un hétéroatome sélectionné à partir du groupe constitué par l'azote, l'oxygène et le soufre pour former un cycle à 5 ou 6 chaînons;
R₁₂ signifie une liaison ou un groupe alkylène inférieur; et
L₁ et L₂ sont identiques ou différents et chacun est de l'hydrogène ou un groupe alkyle inférieur ou L₁ et L₂ sont combinés pour former un groupe alkylène, ou d'un sel pharmaceutiquement acceptable de celui-ci.

2. Utilisation selon la revendication 1 d'un composé de formule (II), dans laquelle le composé est la 5-[4-[2-(5-éthylpyridin-2-yl)éthoxy]benzyl]thiadiazolidine-2,4-dione (pioglitazone).

3. Utilisation selon la revendication 1 d'un composé de formule (II), dans laquelle le composé est la 5-[4-[(1-méthylcyclohexyl)méthoxy]benzyl]thiadiazolidine-2,4-dione (ciglitazone).
